(19) Europäisches Patentamt
European Patent Office
Office européen des brevets

(11) Veröffentlichungsnummer: **0 009 188**
**B1**

# (12) EUROPÄISCHE PATENTSCHRIFT

(45) Veröffentlichungstag der Patentschrift: **16.09.81**

(51) Int. Cl.[3]: **C 07 D 215/22** // C07D215/40, C09B29/08

(21) Anmeldenummer: **79103343.4**

(22) Anmeldetag: **07.09.79**

## (54) 8-Chlor-5,6,7,8-tetrahydro-2-chinolon und 8-Brom-5,6,7,8-tetrahydro-2-chinolon, deren Hydrochlorid bzw. Hydrobromid und Verfahren zu deren Herstellung.

(30) Priorität: **16.09.78 DE 2840437**

(43) Veröffentlichungstag der Anmeldung: **02.04.80 Patentblatt 80/7**

(45) Bekanntmachung des Hinweises auf die Patenterteilung: **16.09.81 Patentblatt 81/37**

(84) Benannte Vertragsstaaten: **BE CH DE FR GB IT NL**

(56) Entgegenhaltungen: **Keine**

(73) Patentinhaber: **HOECHST AKTIENGESELLSCHAFT, Zentrale Patentabteilung Postfach 80 03 20, D-6230 Frankfurt/Main 80 (DE)**

(72) Erfinder: **Meldert, Helmut, Dr., Griesheimer Stadtweg 11, D-6230 Frankfurt am Main 80 (DE)**
Erfinder: **Müller, Werner Heinrich, Dr., Taunusblick 5, D-6239 Eppstein/Taunus (DE)**
Erfinder: **Pressler, Wilfried, Dr., Am Flachsland 15, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# 8-Chlor-5,6,7,8-tetrahydro-2-chinolon und 8-Brom-5,6,7,8-tetrahydro-2-chinolon, deren Hydrochlorid bzw. Hydrobromid und Verfahren zu deren Herstellung

Gegenstand dieser Erfindung sind 8-Clor-5,6,7,8-tetrahydro-2-chinolon und 8-Brom-5,6,7,8-tetrahydro-2-chinolon der Formel

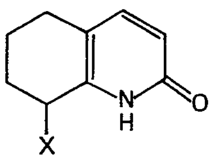

X = Cl bzw. Br

und deren Hydrochlorid bzw. Hydrobromid, die in der Literatur bisher nicht beschrieben sind.

Ein weiterer Gegenstand dieser Erfindung ist ein Verfahren zur Herstellung von 8-Chlor-5,6,7,8-tetrahydro-2-chinolon-hydrochlorid oder 8-Brom-5,6,7,8-tetrahydro-2-chinolon-hydrobromid, das dadurch gekenntzeichnet ist, dass man 3,4,5,6,7,8-Hexahydro-2-chinolon in einem inerten Lösungsmittel bei Temperaturen zwischen 10 und 50°C mit Chlor bzw. Brom umsetzt und die Reaktionslösung nach Beendigung der Halogenzugabe auf Temperaturen zwischen 60 und 80°C erwärmt. Vorzugsweise wird die Umsetzung mit Chlor bzw. Brom bei 25 bis 40°C durchgeführt. Nach Beendigung der Halogenzugabe wird vorzugsweise auf 60 bis 70°C erwärmt.

Die Reaktion verläuft gemäss folgender Bruttogleichung:

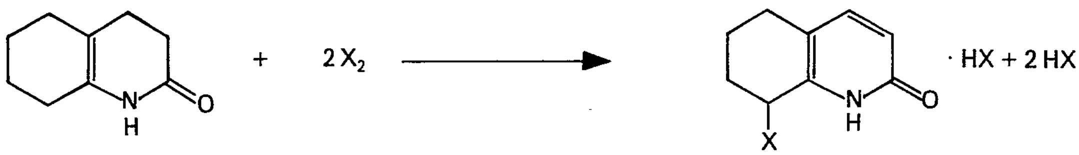


X = Cl bzw. Br

Dieses Ergebnis ist überraschend, da das Verfahren einstufig abläuft, obwohl es formal zwei Reaktionsschritte umfasst, nämlich die halogenierende Dehydrierung in 3,4-Stellung und die Halogenierung in 8-Stellung.

Zur Isolierung der freien Basen genügt es z.B., das Hydrohalogenid in einem inerten Lösungsmittel wie Xylol, Dibutylether, Monochlorbenzol auf etwa 120 bis 150°C zu erhitzen, wobei Halogenwasserstoff entweicht und das freie 8-Chlor-5,6,7,8-tetrahydro-2-chinolon (Fp. 192°C) bzw. 8-Brom-5,6,7,8-tetrahydro-2-chinolon (Fp. 166°C) beim Erkalten der Lösung kristallisiert.

Die erfindungsgemässen neuen Verbindungen sind somit in nur einem Verfahrensschritt aus 3,4,5,6,7,8-Hexahydro-2-chinolon zugänglich geworden, welches seinerseits aus Cyclohexanon und Acrylnitril einfach hergestellt werden kann (vgl. J. Org. Chem. 29, 2781 (1964)).

Die erfindungsgemässen Verbindungen sind sehr reaktive Halogenverbindungen und als solche Schlüsselprodukte für eine Vielzahl von Folgeprodukten. So führt die Umsetzung mit Arylaminen in die Reihe der 8-Arylamino-5,6,7,8-tetrahydro-2-chinolone. Diese aromatischen Amine sind aufgrund ihres hohen Schmelzpunktes (z.B. schmilzt 8-Anilino-5,6,7,8-tetrahydro-2-chinolon bei 225°C) hervorragende Kupplungskomponenten für die Herstellung von Polyesterfarbstoffen.

Bei der erfindungsgemässen Reaktion werden pro Mol Ausgangsverbindung vorzugsweise 1-4 Mol Halogen eingesetzt, insbesondere 2-3 Mol. Ein höherer Halogenüberschuss hat jedoch keinen nachteiligen Einflus auf die Ausbeute an Reaktionsprodukt.

Als Lösungsmittel für die zu halogenierende Ausgangsverbindung kommen prinzipiell reaktionsinerte Lösungsmittel wie chlorierte Kohlenwasserstoffe, Eisessig, Dimethylformamid in Frage. Aus Gründen der optimalen Abtrennung des Reaktionsproduktes sind jedoch solche Lösungsmittel bevorzugt, in denen das Endprodukt wenig löslich ist, beispielsweise chlorierte Kohlenwasserstoffe, wie 1,2-Dichlorethan, Methylenchlorid, Chloroform oder Tetrachlorkohlenstoff.

Hiervon hat sich 1,2-Dichlorethan als besonders vorteilhaft erwiesen, da bei dessen Verwendung die erfindungsgemässen Hydrohalogenide direkt in besonderer Reinheit anfallen.

Die Konzentration der Ausgangsverbindung im Lösungsmittel wird im allgemeinen so gewählt, dass ein Gewichtsteil Ausgangsverbindung in 5 bis 12 Volumenteilen, vorzugsweise 8 bis 10 Volumenteilen des entsprechenden Lösungsmittels gelöst wird.

Weiterhin wurde gefunden, dass es für die erfindungsgemässe Halogenierungsreaktion vorteilhaft ist, die Höhe der Chloreinleitgeschwindigkeit bzw. Bromeintropfgeschwindigkeit in relativ engen Grenzen zu halten. Vorzugsweise wählt man pro 1 Mol an gelöster Ausgansverbindung eine Chloreinleit- bzw. Bromeintropfgeschwindigkeit von 0,2 bis 0,6 Mol Halogen pro Stunde, insbesondere 0,3 bis 0,5 Mol Halogen pro Stunde. Das bedeutet, dass z.B. für 5 Mol Ausgangsverbindung 1 bis 3 Mol Halogen pro Stunde bevorzugt sind.

Während hierbei das Chlor im allgemeinen unverdünnt, d.h. ein Chlorstrom direkt in die Lösung der Ausgangsverbindung eingeleitet wird, ist es zweckmässig, das Brom mit dem zur Anwendung kommenden Lösungsmittel vorher zu verdünnen, und diese Lösung dann gemäss den oben angegebenen Geschwindigkeiten in die Lösung der

Ausgangsverbindung einzutropfen. Die Konzentration der Bromlösung wird im allgemeinen so gewählt, das elementares Brom mit dem 6 bis 12-fachen, vorzugsweise mit dem 8 bis 10-fachen Volumen des für die Reaktion verwendeten Lösungsmittels verdünnt wird.

Während der erfindungsgemässen Nacherwärmung auf 60 bis 80°C scheidet sich das 8-Chlor-5,6,7,8-tetrahydro-2-chinolon-hydrochlorid bzw. das 8-Brom-5,6,7,8-tetrahydro-2-chinolon-hydrobromid in kristalliner Form aus der Lösung ab.

Dies ist ein besonderer Vorteil des erfindungsgemässen Verfahrens, da jeglicher Aufwand für die Abtrennung von Nebenprodukten entfällt und so die erfindungsgemässen Verbindungen auf einfache Weise isoliert werden können.

Versuchsbericht: Weiterverarbeitung von 8-Chlor-5,6,7,8-tetrahydro-2-chinolon-hydrochlorid zu einem Farbstoff

a) Herstellung von 8-Anilino-5,6,7,8-tetrahydro-2-chinolon. 110 g (0,5 Mol) 8-Chlor-5,6,7,8-tetrahydro-2-chinolon-hydrochlorid werden in 800 ml Isopropanol suspendiert und bei 80°C werden 186 g (2,0 Mol) Anilin hinzugetropft, wobei allmählich eine klare Lösung entsteht, die anschliessend noch 10 Minuten unter Rückfluss erhitzt wird. Das beim Erkalten ausfallende farblose, kristalline 8-Anilino-5,6,7,8-tetrahydro-2-chinolon wird schliesslich abgesaugt, mit Acetonitril gewaschen und in einer Ausbeute von 94 g (78,3% der Theorie) isoliert. Es schmilzt bei 225°C.

b) Kupplung von 8-Anilino-5,6,7,8-tetrahydro-2-chinolon. 86 g 2-Chlor-4-nitro-anilin werden in eine Mischung von 175 g konzentrierter Salzsäure und 1000 ml Wasser eingetragen, dann wird so lange gerührt, bis eine homogene Suspension erreicht ist. Nach Abkühlung auf 10°C werden 35 g Natriumnitrit hinzugefügt und die Mischung wird noch 45 Minuten bei 10°C weitergerührt. Die Lösung des Diazoniumsalzes wird dann filtriert, auf 0 bis 5°C abgekühlt und in eine Lösung von 120 g 8-Anilino-5,6,7,8-tetrahydro-2-chinolon in verdünnter Säure und Eis (236 g konzentrierte Salzsäure und 500 g Wasser mit Eis) eingerührt. Dann wird das Gemisch zunächst mit Natronlauge und darauf mit Natriumacetat auf pH 5,5 bis 6 abgestumpft und der ausgefallene Niederschlag abgesaugt, gewaschen und getrocknet.

Der entstandene Farbstoff, 202 g, entspricht der Formel

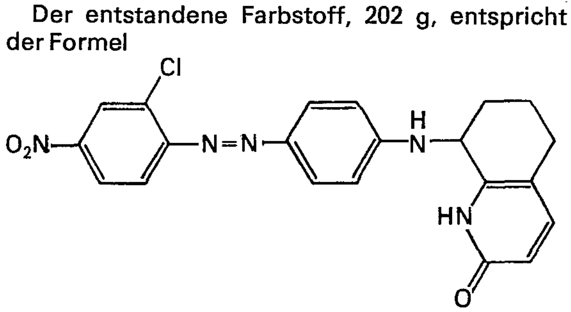

In organischen Lösungsmitteln löst sich das rote Produkt mit oranger Farbe. In feine Verteilung gebracht, färbt der Farbstoff synthetische Polyethylenterephthalatgewebe in orangen Farbtönen mit guter Sublimier-, Wasch- und Lichtechtheit.

Beispiel 1

In einem 1-Liter-Kolben werden 75,5 g (0,5 Mol) 3,4,5,6,7,8-Hexahydro-2-chinolon (Fp. 147°C) in 400 ml 1,2-Dichlorethan gelöst und bei 33 bis 35°C unter Rühren 5 Liter Chlor pro Stunde in die Lösung geleitet, bis nach 4 Stunden die Aufnahme der stöchiometrischen Chlormenge erfolgt ist.

Zur Entfernung des Chlorwasserstoffs wird die Temperatur dann allmählich auf 60°C erhöht und dann noch 10 Minuten bei dieser Temperatur gehalten, bis der Gasabgang beendet ist.

Inzwischen hat sich kristallines Reaktionsprodukt aus der Lösung abgeschieden. Zur Entfernung von noch gelöstem Chlorwasserstoff wird ein trockener Luftstrom duch die Suspension geleitet, bis diese auf 25 bis 30°C abgekühlt ist. Die farblosen Kristalle werden bei 15°C abgesaugt, mit Acetonitril gewaschen, und 68 g 8-Chlor-5,6,7,8-tetrahydro-2-chinolon-hydrochlorid (Fp. 192°C), entsprechend einer Ausbeute von 62% der Theorie werden isoliert.

Beispiel 2

In einem 500 ml-Kolben werden 15,1 g (0,1 Mol) 3,4,5,6,7,8-Hexahydro-2-chinolon in 150 ml 1,2-Dichlorethan gelöst, und bei 35°C wird eine Lösung von 32 g Brom in 100 ml 1,2-Dichlorethan im Laufe von 5 Stunden zugetropft.

Zur Entfernung von Bromwasserstoff wird analog zu Beispiel 1 erwärmt.

Das ausgeschiedene farblose Kristallisat wird schliesslich abgesaugt, mit Acetonitril gewaschen, und 14,5 g 8-Brom-5,6,7,8-tetrahydro-2-chinolon-hydrobromid (Fp. 160°C), entsprechend einer Ausbeute von 47% der Theorie werden isoliert.

Beispiel 3

In einem 2-Liter-Kolben werden 151 g (1,0 Mol) 3,4,5,6,7,8-Hexahydro-2-chinolon in 800 ml 1,2-Dichlorethan gelöst, und bei 34–35°C werden unter Rühren 6,5 Liter Chlor pro Stunde in die Lösung geleitet, bis nach 7 Stunden die Aufnahme der stöchiometrischen Chlormenge erfolgt ist.

Nun wird analog zu Beispiel 1 weitergearbeitet und schliesslich 154 g 8-Chlor-5,6,7,8-tetrahydro-2-chinolon-hydrochlorid, entsprechend einer Ausbeute von 70% der Theorie isoliert

**Patentansprüche:**

1. 8-Chlor-5,6,7,8-tetrahydro-2-chinolon sowie dessen Hydrochlorid und 8-Brom-5,6,7,8-tetrahydro-2-chinolon sowie dessen Hydrobromid der Formeln

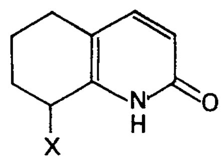

sowie

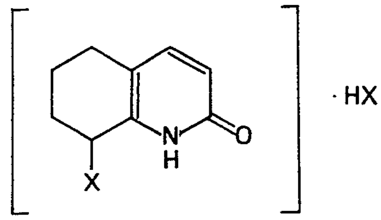

(I) (II)

mit X=Cl bzw. Br.

2. Verfahren zur Herstellung von 8-Chlor-5,6,7,8-tetrahydro-2-chinolon sowie dessen Hydrochlorid und 8-Brom-5,6,7,8-tetrahydro-2-chinolon sowie dessen Hydrobromid der Formeln

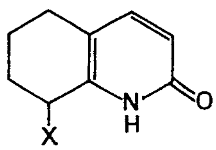

sowie

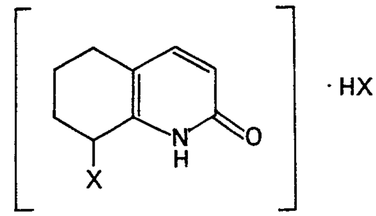

(I) (II)

mit X=Cl bzw. Br.

dadurch gekennzeichnet, dass man 3,4,5,6,7,8-Hexahydro-2-chinolon in einem inerten Lösungsmittel bei Temperaturen zwischen 10 und 50°C mit Chlor bzw. Brom umsetzt und die Reaktionslösung nach Beendigung der Halogenzugabe auf Temperaturen zwischen 60 und 80°C erwärmt, wobei die Verbindung der Formel (II) mit X=Cl bzw. Br entsteht, und gegebenenfalls anschliessend diese Verbindung in einem inerten Lösungsmittel erhitzt und nach Entweichen des Halogenwasserstoffs die Lösung abkühlen lässt, wobei die Verbindung der Formel (I) mit X=Cl bzw. Br. auskristallisiert.

3. Verfahren nach Anspruch 2, dadurch gekennzeichnet, dass man die Umsetzung mit Chlor bzw. Brom bei 25 bis 40°C durchführt.

4. Verfahren nach einem der Ansprüche 2 und 3, dadurch gekennzeichnet, dass man die Reaktionslösung nach Beendigung der Halogenzugabe auf 60 bis 70°C erwärmt.

5. Verfahren nach einem der Ansprüche 2 bis 4, dadurch gekennzeichnet, dass man als inertes Lösungsmittel einen chlorierten Kohlenwasserstoff verwendet.

6. Verfahren nach einem der Ansprüche 2 bis 4, dadurch gekennzeichnet, dass man als inertes Lösungsmittel 1,2-Dichlorethan verwendet.

7. Verfahren nach einem der Ansprüche 2 bis 6, dadurch gekennzeichnet, dass man pro 1 Mol an 3,4,5,6,7,8-Hexahydro-2-chinolon 0,3 bis 0,5 Mol Halogen pro Stunde einsetzt.

8. Verfahren nach einem der Ansprüche 2 bis 7, dadurch gekennzeichnet, dass man pro 1 Mol an 3,4,5,6,7,8-Hexahydro-2-chinolon insgesamt 1 bis 4 Mol Halogen einsetzt.

9. Verfahren nach einem der Ansprüche 2 bis 8, dadurch gekennzeichnet, dass Brom mit dem 6 bis 12-fachen Volumen des für die Reaktion verwendeten inerten Lösungsmittels verdünnt eingesetzt wird.

**Revendications**

1.-Chloro-8 tétrahydro-5,6,7,8 quinolone-2 ainsi que son chlorhydrate, et bromo-8 tétrahydro-5,6,7,8 quinolone-2 ainsi que son bromhydrate, qui répondent aux formules

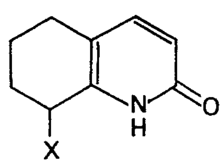

et

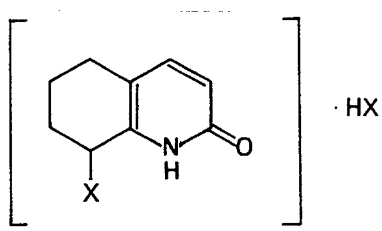

(I) (II)

où X représente le chlore ou le brome.

2.- Procédé de préparation de la chloro-8 tétrahydro-5,6,7,8 quinolone-2 ainsi que de son chlorhydrate, et de la bromo-8 tétrahydro-5,6,7,8 quinolone-2 ainsi que de son bromhydrate, qui répondent aux formules

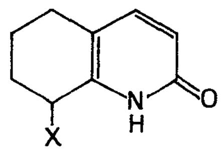

(I)

et

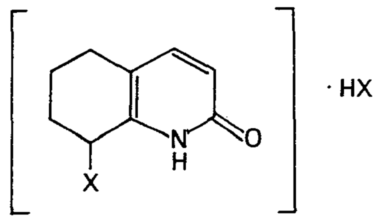

(II)

dans lesquelles X représente Cl ou Br, procédé caractérisé en ce qu'on fait réagir l'hexahydro-3,4,5,6,7,8 quinolone-2, dans un solvant inerte, à une température comprise entre 10 et 50 °C, respectivement avec le chlore ou le brome et, lorsque l'addition d'halogène est terminée, on chauffe la solution réactionnelle à une température comprise entre 60 et 80 °C, d'où formation du composé (II) avec X=Cl ou Br, puis, le cas échéant, on chauffe ce composé dans un solvant inerte et, après dégagement de l'halogénure d'hydrongène, on laisse refroidir la solution, ce qui entraîne la cristallisation du composé (I) avec X = Cl ou Br.

3.- Procédé selon la revendication 2, caractérisé en ce qu'on effectue la réaction avec le chlore ou le brome à une température 25 à 40 °C.

4.- Procédé selon l'une des revendications 2 et 3, caractérisé en ce que la solution réactionnelle est chauffée, après la fin de l'addition de l'halogène, à une température de 60 à 70 °C.

5.- Procédé selon l'une quelconque des revendications 2 à 4, caractérisé en ce qu'on utilise comme solvant inerte un hydrocarbure chloré.

6.- Procédé selon l'une quelconque des revendications 2 à 4, caractérisé en ce qu'on utilise le dichloro-1,2 éthane comme solvent inerte.

7.- Procédé selon l'une quelconque des revendications 2 à 6, caractérisé en ce qu'on met en jeu, pour 1 mole d'hexahydro-3,4,5,6,7,8 quinolone-2, de 0,3 à 0,5 mole d'halogène par heure.

8.- Procédé selon l'une quelconque des revendications 2 à 7, caractérisé en ce qu'on met en jeu, pour 1 mole d'hexahydro-3,4,5,6,7,8 quinolone-2, de 1 à 4 moles d'halogène au total.

9.- Procédé selon l'une quelconque des revendications 2 à 8, caractérisé en ce que le brome que l'on met en jeu est dilué avec de 6 à 12 fois le volume du solvant inerte utilisé pour la réaction.

**Claims:**

1. 8-chloro-5,6,7,8-tetrahydro-2-quinolone as well as its hydrochloride and 8-bromo-5,6,7,8-tetrahydro-2-quinolone as well as its hydrobromide of the formulae

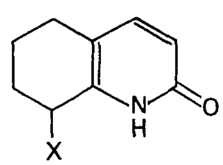

(I)

as well as

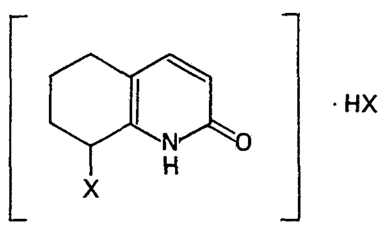

(II)

wherein X=Cl or Br.

2. Process for the manufacture of 8-chloro-5,6,7,8-tetrahydro-2-quinolone as well as its hydrochloride and 8-bromo-5,6,7,8-tetrahydro-2-quinolone as well as its hydrobromide of the formulae

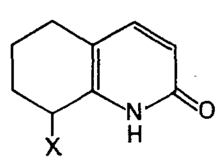

(I)

as well as

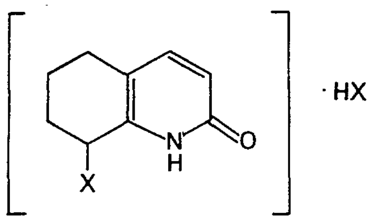

(II)

wherein X=Cl or Br.

characterized by reacting 3,4,5,6,7,8-hexahydro-2-quinolone with chlorine or bromine in an inert solvent and at a temperature of from 10 to 50 °C and heating the reaction solution to 60 to 80 °C after termination of the halogen addition, whereupon the compound of formula (II) with X=Cl or Br, respectively, is formed and optionally heating said compound subsequently in an inert solvent and allowing the solution to cool after the hydrogen halide has escaped, whereupon the compound of formula (I) with X=Cl or Br, respectively, is formed.

3. The process claimed in claim 2, wherein the reaction with chlorine or bromine is effected at a temperature of from 25 to 40 °C.

4. The process claimed in claim 2 or 3 wherein, after termination of the halogen addition, the reaction solution is heated to 60 to 70 °C.

5. The process of any of claims 2 to 4, wherein the inert solvent is a chlorinated hydrocarbon.

6. The process of any of claims 2 to 4, wherein the inert solvent is 1,2-dichloroethane.

7. The process of any of claims 2 to 6, wherein 0.3 to 0.5 mol of halogen per hour is used for each mol of 3,4,5,6,7,8-hexahydro-2-quinolone.

8. The process of any of claims 2 to 7, wherein 1 to 4 mols of halogen are used altogether for each mol of 3,4,5,6,7,8-hexahydro-2-quinolone.

9. The process of any of claims 2 to 8, wherein the bromine is diluted with 6 to12 times the volume of the inert solvent used for the reaction.